# EUROPEAN PATENT SPECIFICATION

(11) **EP 2 766 051 B1**
(45) Date of publication and mention of the grant of the patent: **04.12.2019**
(21) Application number: 12840663.4
(22) Date of filing: 11.10.2012
(51) Int. Cl.: A61K 49/22, A61K 9/16, A61K 49/04

(54) **PORPHYRIN-PHOSPHOLIPID CONJUGATE MICROBUBBLES AND THEIR USE AS CONTRAST AGENTS.**
PORPHYRIN-PHOSPHOLIPIDKONJUGAT-MIKROBLÄSCHEN UND IHRE VERWENDUNG ALS KONTRASTMITTEL
MICROBULLES DE CONJUGUÉ PORPHYRINE-PHOSPHOLIPIDE ET LEUR UTILISATION COMME AGENTS DE CONTRASTE

(30) Priority: 13.10.2011 US 201161546663 P
(43) Date of publication of application: 20.08.2014
(73) Proprietor: University Health Network, Toronto, ON M5G 2C4 (CA)
(72) Inventor: ZHENG, Gang, Toronto, Ontario M2N 4M8 (CA); LOVELL, Jonathan, Toronto, Ontario M4Y 1R5 (CA); HUYNH, Elizabeth, Mississauga, Ontario L5W 1V3 (CA)
(74) Representative: Carpmaels & Ransford LLP
(86) International application number: PCT/CA2012/000937
(87) International publication number: WO 2013/053042

(56) References cited:
- WO-A1-2011/044671
- US-A- 6 123 923
- Elizabeth Huynh ET AL: "Porphyrin shell microbubbles with intrinsic ultrasound and photoacoustic properties", Journal of the American Chemical Society, 10 October 2012 (2012-10-10), page 16464, XP055186875, United States DOI: 10.1021/ja305988f Retrieved from the Internet: URL:http://www.ncbi.nlm.nih.gov/pubmed/228 27774 [retrieved on 2015-04-30]
- BORDEN M A ET AL: "Physico-chemical properties of the microbubble lipid shell: Composition, microstructure & properties of targeted ultrasound contrast agents", PROCEEDINGS - IEEE ULTRASONICS SYMPOSIUM - PROCEEDINGS - 2004 IEEE ULTRASONICS SYMPOSIUM: A CONFERENCE OF THE IEEE INTERNATIONAL ULTRASONICS, FERROELECTRICS, AND FREQUENCY CONTROL SOCIETY, UFFC-S 2004 INSTITUTE OF ELECTRICAL AND ELECTRONICS ENGINEERS, vol. 1, 2004, pages 20-23, XP002739132,
- JAMES J. KWAN ET AL: "Lipid monolayer collapse and microbubble stability", ADVANCES IN COLLOID AND INTERFACE SCIENCE, vol. 183-184, 1 November 2012 (2012-11-01) , pages 82-99, XP055186840, ISSN: 0001-8686, DOI: 10.1016/j.cis.2012.08.005
- ELIZABETH HUYNH ET AL: "In situ conversion of porphyrin microbubbles to nanoparticles for multimodality imaging", NATURE NANOTECHNOLOGY, vol. 10, no. 4, 30 March 2015 (2015-03-30) , pages 325-332, XP055186876, ISSN: 1748-3387, DOI: 10.1038/nnano.2015.25
- M AHLERS ET AL: "Quenching of fluorescein-conjugated lipids by antibodies", BIOPHYS J, vol. 63, 1 January 1992 (1992-01-01), pages 823-838, XP055187003,
- DE SOUZA: 'One particle to rule them all?' NATURE METHODS vol. 8, no. 5, May 2011, pages 370 - 371, XP055148848
- LOWELL ET AL.: 'Porphysome nanovescicles generated by porphyrin bilayers for use as multimodal biophotonic contrast agents' NATURE MATERIALS vol. 10, April 2011, pages 324 - 332, XP002678939

## Description

### FIELD OF THE INVENTION

This invention relates to the field of microbubbles and, more specifically, to microbubbles formed from porphyrin conjugated to a phospholipid side chain, as defined in the claims.

### BACKGROUND OF THE INVENTION

Microbubbles are gas-filled microspheres which confer acoustic contrast for ultrasound imaging. Currently, microbubbles are routinely used in the clinic for cardiac diagnosis using echocardiography [1] and are being used for diagnosing cancers and guiding treatment [2], [3]. Ultrasound is one of the most affordable and accessible imaging modalities and therefore any new enabling ultrasound contrast technology holds potential for rapid and widespread impact with existing clinical infrastructure. The expanding field of microbubble research extends beyond diagnostic applications. Preclinical studies are using targeted microbubbles in molecular imaging for assessing pathophysiology [4] as well as in ultrasound-triggered drug and gene delivery [5], [6] for treatment of various diseases and crossing of the blood brain barrier [7], [8].

Clinically used microbubbles are typically formed from fluorinated gases incorporated into a self-assembled phospholipid shell. As such, fluorophores can easily be incorporated into the microbubble shell to verify binding of the microbubble to the target region [9] or for evidence of disruption of the microbubble upon bursting with ultrasound [10]. However, the effect of the presence of these molecules on the stability of the microbubble shell has not been widely investigated and the total fraction of fluorophore relative to the rest of the shell has been minimal. Previously, we introduced a new paradigm for forming a phospholipid bilayer-like structure entirely from porphyrin-conjugated phospholipids [11]. Due to the high porphyrin character, these structures demonstrated unique physical properties, as well as robust intrinsic multimodal imaging properties suitable for fluorescence, photoacoustic, MR and PET.

Borden M A et al ("Physico-chemical properties of the microbubble lipid shell", Proceedings - IEEE Ultrasonics symposium - Proceedings - 2004 IEEE Ultrasonics symposium: A conference of the IEEE international ultrasonics, ferroelectrocs, and frequency control society, UFFC-S 2004 Institute of electrical and electronics engineers, vol. 1, 2004, pages 20-23) describes the composition, microstructure and properties of certain targeted microbubbles.

M Ahlers et al ("Quenching of fluorescein-conjugated lipids by antibodies", Biophys J, vol. 63, 1 January 1992, pages 823-838) describes the use of model biomembrane systems, monolayers, micelles and vesicles to study the influence of chemical and physical variables of hapten presentation at membrane interfaces on antibody binding.

US 6 123 923 A describes optoacoustic contrast agents and methods of diagnostic and therapeutic imaging using optoacoustic contrast agents.

Lovell et al. ("Porphysome nanovescicles generated by porphyrin bilayers for use as multimodal biophotonic contrast agents", Nature Materials, vol. 10, April 2011, pages 324-332) describes the development and photothermal and photoacoustic properties of certain porphysomes.

De Souza ("One particle to rule them all?" Nature Methods, vol. 8, no. 5, May 2011, pages 370-371) describes the genesis and properties of porphysomes.

### SUMMARY OF THE INVENTION

The invention provides a monolayer microbubble with a gas encapsulated therein, the monolayer comprising porphyrin-phospholipid conjugate, a second phospholipid and a PEGylated emulsifier, wherein
the porphyrin-phospholipid conjugate comprises one porphyrin, porphyrin derivative or porphyrin analog covalently attached to a lipid side chain by a carbon chain linker of 0 to 20 carbons, preferably at the sn-1 or the sn-2 position, of one phospholipid;
the porphyrin-phospholipid conjugate present at up to 40 molar % of the monolayer;
the porphyrin, porphyrin derivative or porphyrin analog in the porphyrin-phospholipid conjugate is selected from the group consisting of hematoporphyrin, protoporphyrin, tetraphenylporphyrin, a pyropheophorbide, a bacteriochlorophyll, chlorophyll a, a benzoporphyrin, a tetrahydroxyphenyl chlorin, a purpurin, a benzochlorin, a naphthochlorins, a verdin, a rhodin, a keto chlorin, an azachlorin, a bacteriochlorin, a tolyporphyrin, a benzobacteriochlorin, texaphyrin, a sapphyrin, hexaphyrin, porphycene, inverted porphyrin, phthalocyanine, and naphthalocyanine; and
the gas is a biologically inert gas, a fluorinated gas, or selected from the group consisting of air, 02, N2, H2, CO2, N2O, noble gases, hydrocarbon gases, perfluorocarbon, sulphur hexafluoride, perfluoromethane, perfluoroethane, perfluoropropane, perfluorobutane and combinations thereof. In a further aspect, there is provided a method of preparing microbubbles of the invention, comprising mixing a gas, a porphyrin-phospholipid conjugate and another phospholipid, wherein the porphyrin-phospholipid conjugate comprises one porphyrin, porphyrin derivative or porphyrin analog covalently attached to a lipid side chain, preferably at the sn-1 or the sn-2 position, of one phospholipid.

In a further aspect, there is provided a method of performing ultrasound imaging on a target area in a subject comprising providing the microbubble described herein; administering the microbubble to the subject; and imaging the target area using ultrasound.

In a further aspect, there is provided a method of imaging a target area in a subject, comprising providing the microbubble described herein; administering the microbubble to the subject; and measuring and/or detecting the photoacoustic signal at the target area. Preferably, the method further comprises bursting the microbubble at the target area prior to measuring and/or detecting the increase in photoacoustic signal.

### BRIEF DESCRIPTION OF THE DRAWINGS

Embodiments of the invention may best be understood by referring to the following description and accompanying drawings. In the drawings:

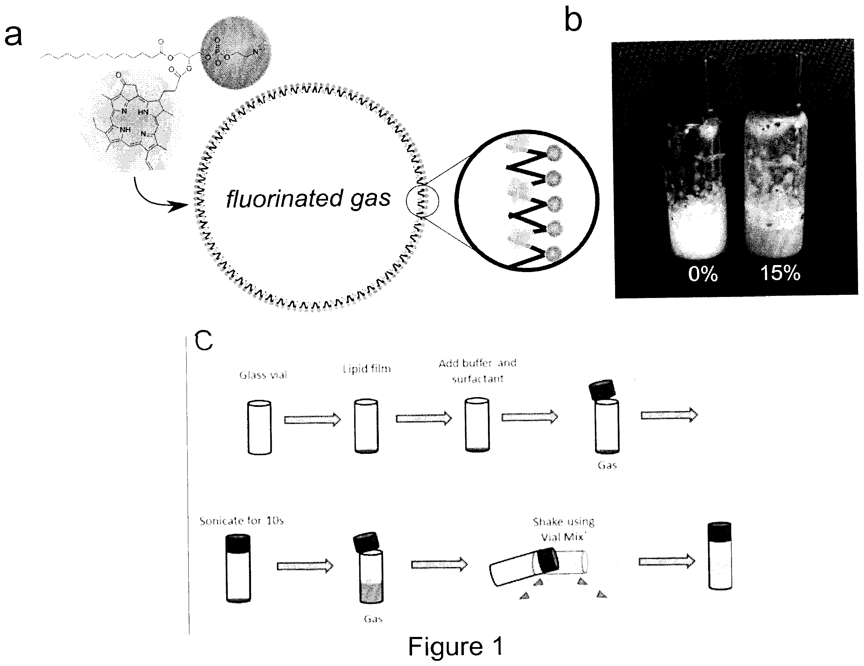

**Figure 1** shows that porphyrin-shell ("**porshe**") microbubbles can be formed easily. A) Schematic representation of porphyrin-lipid (top) and porshe microbubbles formed from them encapsulating gas (bottom). B). Image of microbubbles formed with 15 mol% porphyrin-lipid (pyropheophorbide-lipid). C) Straightforward one-pot synthesis to generate porshe microubbles using standard microbubble procedures.

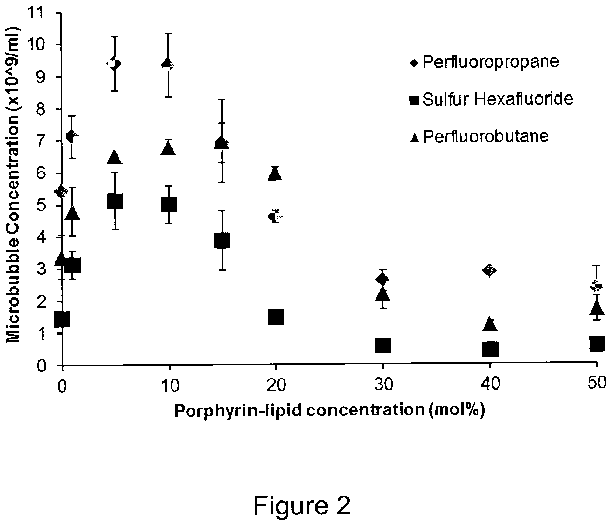

**Figure 2** shows that incorporation of porphyrin-lipid improves yield of microbubble formation using DSPC, PEG40S and a 0.01M PBS buffer. The number of microbubbles was counted as a function of the amount of porphyrin-lipid included in the formulation. Microbubbles were formed from three commonly used fluorinated gases: perfluoropropane (PFP), perfluorobutane (PFB) and sulfur hexafluoride (SF6). Inclusion of 5 to 15% porphyrin-lipid results in optimal microbubble formation for this particular mixture of phospholipids, surfactant and buffer.

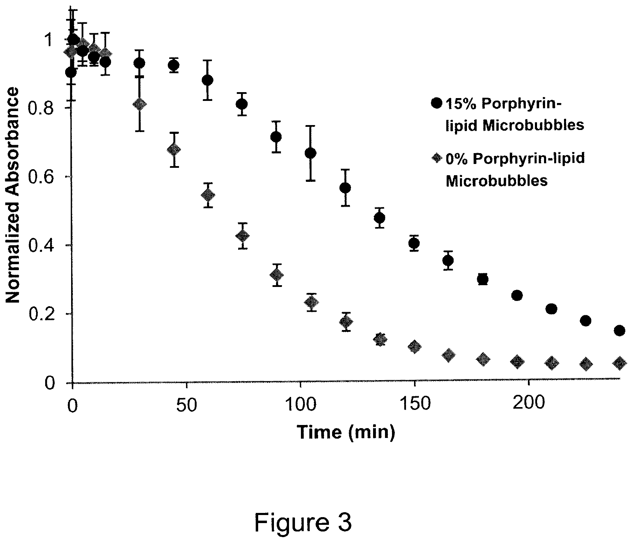

**Figure 3** shows that porshe microbubbles have improved stability in serum. Microbubbles were formed with or without 15 molar % porphyrin-lipid and incubated in bovine serum at 37 C. Absorbance represents the number of remaining microbubbles based on light scattering. Porphyrin microbubbles were more stable in serum with a half-life twice as long as microbubbles formed without porphyrin-lipid.

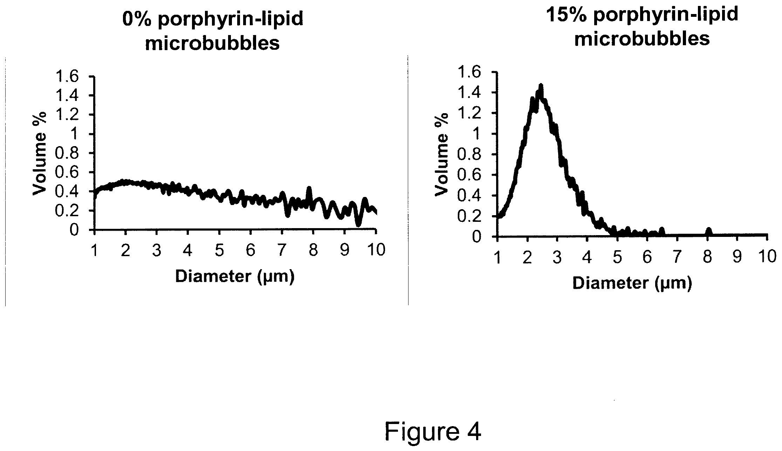

**Figure 4** shows that porphyrin-lipid microbubbles have superior size distribution around 2-3 microns compared with microbubbles formed without porphyrin-lipid. Microbubbles were measured using a Coulter counter. Note the microbubbles formed with porphyrin-lipid have a large and distinct population 2-4 microns in size. Traditional lipid microbubbles often display a polydispersed size distribution.

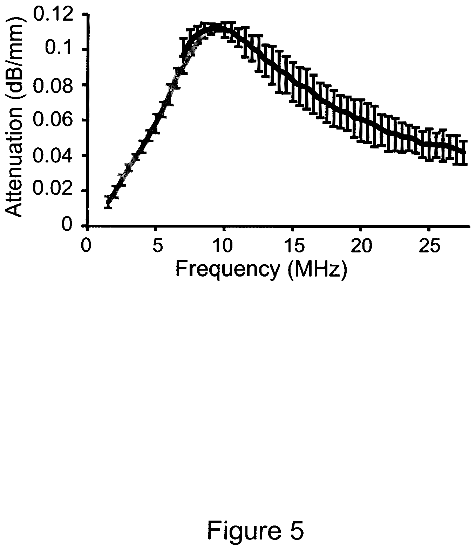

**Figure 5** shows the acoustic attenuation as a function of frequency for porshe microbub-bles. Grey: 1.5-8.5 MHz transducer. Black: 7-27.5 MHz trans-ducer. Porshe microbubbles exhibit a resonance frequency between 9 - 10 MHz.

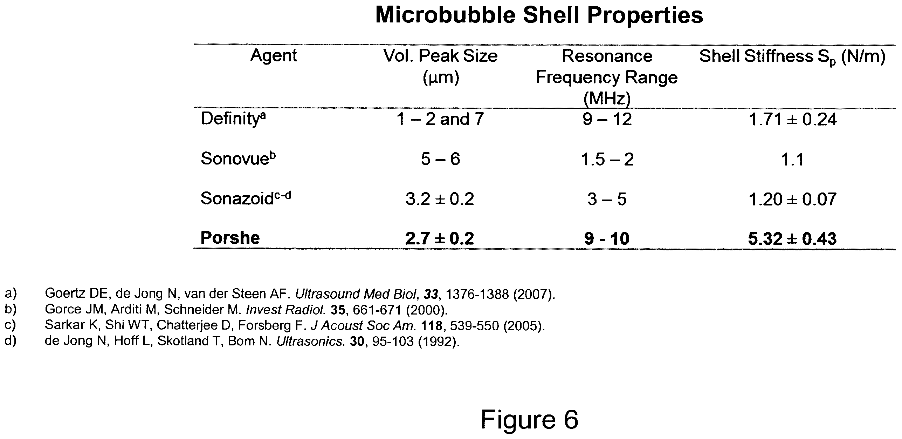

**Figure 6** shows a table of microbubble parameters and shell properties for porshe and commercial lipid microbubbles (literature values). Inclusion of porphyrin-lipid results in an increase in microbubble shell stiffness.

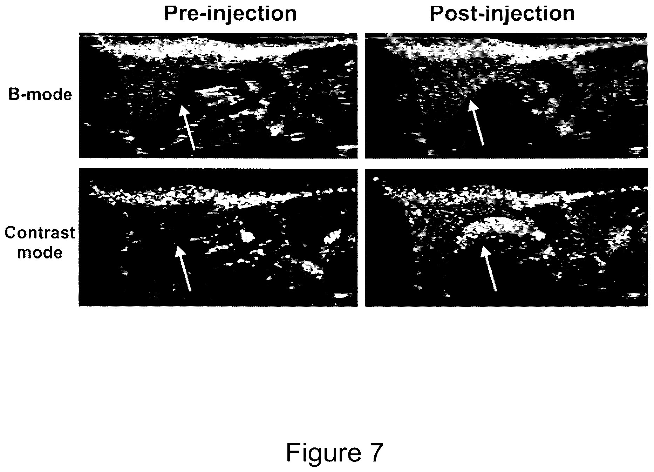

**Figure 7** shows the use of porshe microbubbles for enhanced standard and contrast ultrasound imaging of mouse jugular vein. Porshe microbubbles were injected into a mouse via teil vein injection and both B-mode and contrast mode images were aquired. An 18 MHz transducer frequency was used for imaging.

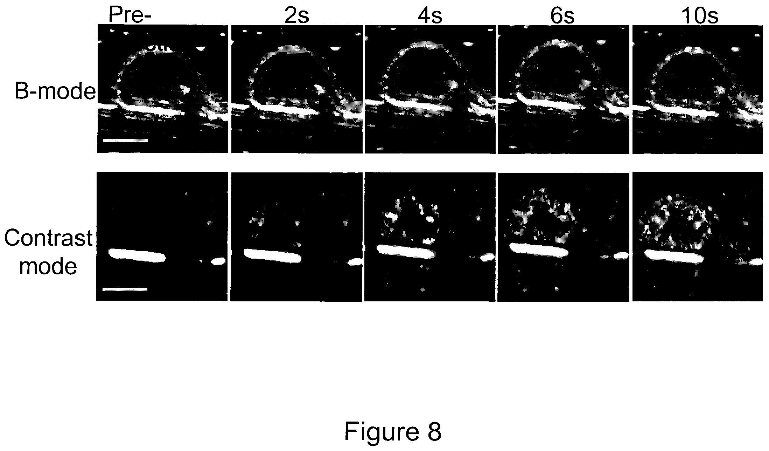

**Figure 8** shows the use of porshe microbubbles for enhanced contrast ultrasound imaging of a subcutaneous breast cancer (MDA-MB-231) tumor xenograft in a mouse. Porshe microbubbles were injected into a mouse via tail vein injection and both B-mode and contrast mode images were aquired. A 5-12MHz transducer frequency was used. 1cm scale bar shown.

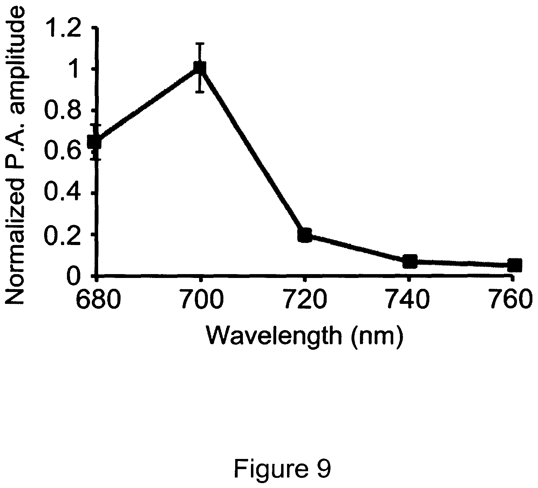

**Figure 9** shows the photoacoustic spectrum of porshe microbubbles (mean from 3 measurements +/- SD) acquired from a 5ns pulsed laser and a 10MHz single element transducer from a solution of porshe microbubbles.

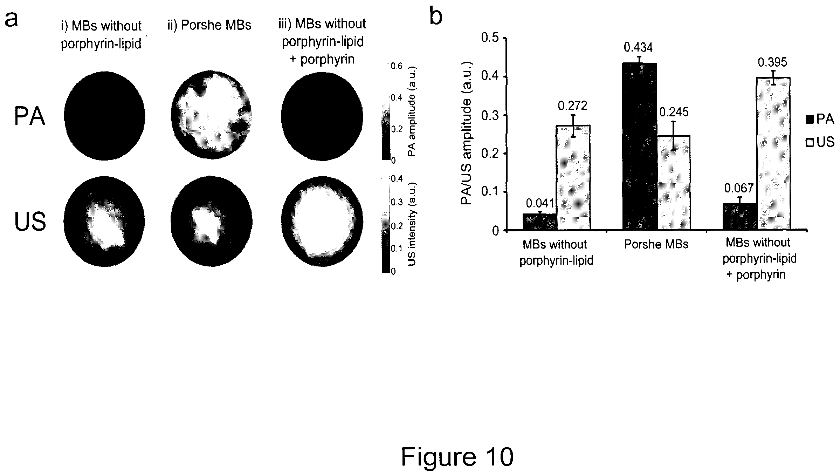

**Figure 10** shows the photoacoustic and ultrasound properties of porshe microbubbles imaged in a cylindrical well. PA (top) and US (bottom) images of microbubbles (MBs) formed without porphyrin-lipid, porshe MBs and MBs without porphyrin-lipid incubated with equimolar pyropheophorbide-α (pyro) in 9 mm diameter wells within a plastic phantom. PA images were acquired using a 700 nm 5 ns pulsed laser and 10 MHz single element transducer.

### DETAILED DESCRIPTION OF THE PREFERRED EMBODIMENTS

Here we describe "porshe" microbubbles, a new class of microbubbles composed with a porphyrin-phospholipid shell encapsulating a gas, preferably a fluorinated gas.

In an aspect, there is provided a microbubble comprising a monolayer with a gas encapsulated therein, the monolayer comprising porphyrin-phospholipid conjugate, wherein the porphyrin-phospholipid conjugate comprises one porphyrin, porphyrin derivative or porphyrin analog covalently attached to a lipid side chain, preferably at the sn-1 or the sn-2 position, of one phospholipid, as defined in the claims.

Examples of porphyrin-phospholipid conjugates used in forming microbubbles of the application are described in WO 11/044671 by the Applicant.

In different embodiments, the porphyrin-phospholipid conjugate may be present at different molar % up to 40 molar %, including 5, 10, 15, 20, 30, and 40 molar%.

The porphyrin, porphyrin derivative or porphyrin analog in the porphyrin-phospholipid conjugate is selected from the group consisting of hematoporphyrin, protoporphyrin, tetraphenylporphyrin, a pyropheophorbide, a bacteriochlorophyll, chlorophyll a, a benzoporphyrin, a tetrahydroxyphenyl chlorin, a purpurin, a benzochlorin, a naphthochlorins, a verdin, a rhodin, a keto chlorin, an azachlorin, a bacteriochlorin, a tolyporphyrin, a benzobacteriochlorin, texaphyrin, a sapphyrin, hexaphyrin, porphycene, inverted porphyrin, phthalocyanine, and naphthalocyanine.

In some embodiments, the phospholipid in the porphyrin-phospholipid conjugate comprises phosphatidylcholine, phosphatidylethanoloamine, phosphatidylserine or phosphatidylinositol. Preferably, the phospholipid comprises an acyl side chain of 12 to 22 carbons.

In some embodiments, the porphyrin in the porphyrin-phospholipid conjugate is pyropheophorbide-a acid.

In some embodiments, the porphyrin in the porphyrin-phospholipid conjugate is a bacteriochlorophyll derivate.

In some embodiments, the phospholipid in the porphyrin-phospholipid conjugate is 1-Palmitoyl-2-Hydroxy-sn-Glycero-3-Phosphocholine or 1-Stearoyl-2-Hydroxy-sn-Gycero-3-Phosphocholine.

In some embodiments, the porphyrin-phospholipid conjugate is pyropheophorbide-lipid.

In some embodiments, the porphyrin-phospholipid conjugate is oxy-bacteriochlorophyll-lipid.

The porphyrin is conjugated to the glycerol group on the phospholipid by a carbon chain linker of 0 to 20 carbons.

The microbubble further comprises a PEGylated emulsifier, which preferably has a molecular weight ranging from 1000 to 5000. In a preferred embodiment, the PEGylated emulsifier is selected from the group consisting of N-(methoxypolyethylene glycol 5000 carbamoyl)-1,2-dipalmitoyl-sn-glycero-3-phosphatidylethanolamine (MPEG5000-DPPE), 1,2-dimyristoyl-sn-glycero-3-phosphoethanolamine-N-[methoxy(polyethylene glycol)-2000 (DMPE-PEG2000), 1,2-distearoyl-sn-glycero-3-phosphoethanolamine- N-[methoxy(polyethylene glycol)-2000 (DSPE-PEG2000), Polyoxyethylene 40 stearate (PEG40S) and combinations thereof. In certain embodiments, the PEG or PEG-lipid is present in an amount of about 10 molar %.

In some embodiments, the microbubble is substantially spherical and between 0.8 µm - 18µm in diameter, preferably, between 1 µm - 9µm in diameter, and further preferably between 2 µm - 4µm in diameter.

In some embodiments, the porphyrin-phospholipid conjugate comprises a metal chelated therein, optionally a radioisotope of a metal. Preferably, the metal is selected from the group consisting of Zn, Cu, Mn, Fe and Pd.

In some embodiments, the encapsulated gas is a biologically inert gas.

Gases suitable for use in microbubbles include: air, O₂, N₂, H₂, CO₂, N₂O, noble gases, hydrocarbon gases, perfluorocarbon, other fluorinated gases and combinations thereof. Fluorinated gases are preferable because they are not soluble in water which results in greater stability of the microbubble in vitro and in vivo. Other examples of insoluble gases that may be used include sulphur hexafluoride, perfluoromethane, perfluoroethane, perfluoropropane, perfluorobutane and combinations thereof.

Preferred phospholipids used in microbubble formulations contain carbon chain lengths varying from 12-24 carbons and headgroups including phosphatidylcholines, phosphatidylethanolamines, phosphatidic acid and phosphatidylglycerols. In a further embodiment, the phospholipid is selected from the group consisting of 1,2-dipalmitoyl-sn-glycero-3-phosphatidic acid (DPPA), 1,2-dipalmitoyl-sn-glycero-3-phosphatidylcholine (DPPC), 1,2-distearoyl-sn-glycero-3-phosphocholine (DSPC), 1,2-dimyristoyl-sn-glycero-3-phosphocholine (DMPC), 1,2-dibehenoyl-sn-glycero-3-phosphocholine (DBPC), 1,2-diarachidoyl-sn-glycero-3-phosphatidylcholine (DAPC), 1,2-dilignoceroyl-sn-glycero-3-phosphatidylcholine(DLgPC), 1,2-dipalmitoyl-sn-glycero-3-[phosphor-rac-(1-glycerol)] (DPPG) and combinations thereof.

The microbubbles can be formed in a solution with a pH buffer and salts of varying type and concentration. Additives may be included in the buffer for increased storage and handling stability. In some embodiments, the microbubble is formed in solution comprising at least one additive selected from the group consisting of Sodium Chloride, Sodium Phosphate, Propylene glycol, Glycerol and Polyethylene glycol (preferably ranging from 1000 to 6000 molecular weight).

In some embodiments, the microbubble further comprises a targeting molecule.

In a further aspect, there is provided a method of preparing microbubbles as defined in the claims, comprising mixing a gas, a porphyrin-phospholipid conjugate and another phospholipid, wherein the porphyrin-phospholipid conjugate comprises one porphyrin, porphyrin derivative or porphyrin analog covalently attached to a lipid side chain, preferably at the sn-1 or the sn-2 position, of one phospholipid.

Preferably, prior to mixing with the gas, the porphyrin-phospholipid conjugate is mixed with the other phospholipid to form a lipid film and then hydrated in buffer.

In a further aspect, there is provided a method of performing ultrasound imaging on a target area in a subject comprising providing the microbubble described herein; administering the microbubble to the subject; and imaging the target area using ultrasound.

In a further aspect, there is provided a method of imaging a target area in a subject, comprising providing the microbubble described herein; administering the microbubble to the subject; and measuring and/or detecting the photoacoustic signal at the target area. Preferably, the method further comprises bursting the microbubble at the target area prior to measuring and/or detecting the photoacoustic signal.

In some embodiment, the imaging is breast imaging, tumour imaging, carotid neovascularisation imaging, or endoscopic imaging.

The following examples are illustrative of various aspects of the invention.

### EXAMPLES

### Methods

### Formation of Porphyrin-lipid Microbubbles

Lipid films were prepared in 12 mm x 35 mm clear glass threaded vials (Fisher Scientific) by combining 1,2-distearoyl-sn-glycero-3-phosphocholine (DSPC, Avanti Polar Lipids) dissolved in chloroform with porphyrin-lipid (pyropheophorbide-lipid). Films were dried under a stream of nitrogen gas and further dried under vacuum for 30 minutes. Lipid films were stored under argon gas at -20°C until hydration with 0.01M phosphate buffered saline (150 mM NaCl, 10 mM phosphate, pH 7.4) and polyoxyethylene-40 stearate (PEG40S, Sigma-Aldrich) dissolved in deionized water. The total lipid concentration was either 0.5mg/ml or 1mg/ml in a 0.5 ml volume. The headspace of each vile was filled with gas. Each vile was placed in a 65°C heated water bath to raise the solution temperature above the transition temperature of the incorporated lipids. The heated vile was sonicated for 10s (Bransonic Model 2510) to disperse the lipid film into solution and gas was added to the headspace once again. Microbubbles were formed by shaking in a Vial-Mix™ (Bristol-Myers Squibb) for 45 s.

### Characterization of concentration and size distribution of microbubbles

Porphyrin-lipid and regular microbubbles were formulated using porphyrin-lipid at the indicated molar %, 10 molar% PEG40S and the remaining DSPC. The headspace of the vial was filled with sulfur hexafluoride (SF₆, Sigma-Aldrich), perfluorobutane (PFB, Fluoromed L.P) or perfluoropropane (PFP, Fluoromed L.P) gas. After activation of the microbubbles via shaking, microbubbles were used within 6 hours. The size distribution and concentration (number of bubbles/ml) of each microbubble formulation was measured using a Coulter counter system (Multisizer III; Beckman Coulter Inc).

Varying volumes of microbubbles were added to 10ml of Isoton-II electrolyte solution (Beckman Coulter Inc) to obtain a microbubble count in the range of 100,000 - 300,000 microbubbles; the dilution was accounted for in the calculation of the microbubble concentration. The number and size distribution were measured using a 30µm aperture which detected microbubbles with diameters in the range of 0.8 µm to 18 µm. For each microbubble formulation, three samples were measured.

### In vitro microbubble stability in serum

Microbubble stability was compared between microbubbles formed with and without 15 molar% porphyrin-lipid in fetal bovine serum (FBS) at 37°C. Microbubbles were incubated with FBS at a concentration of 4.7x10⁸ microbubbles/ml in a 96-well plate. The absorbance was recorded at 580nm with a SpectraMax Plus384 plate reader (Molecular Devices) at 0min, 1min, 5min, 10min, 15min and then at 15minute intervals up to 4 hours after the beginning of incubation at 37°C. The plate was mixed for 5s prior to each measurement. The absorbance is representative of the microbubble concentration. The stability of three samples was measured for each formulation.

### Acoustic characterization of microbubbles

Acoustic characterization was performed with a1 ml volume of porshe microbubbles composed of 15 molar % porphyrin-lipid, 10 molar % PEG40S, 75 molar % DSPC in a 0.5 mg/ml total lipid concentration with PFP gas. After 1 min decantation, the total volume extracted from the bottom of each vial was 0.5 ml using an 18 gauge needle. Following extraction, the agent was placed within a new 1.5 ml vial, topped with PFP gas and loosely sealed with parafilm (Fisher Scientific). The vial was then gently mixed by hand for 10 s prior to Coulter Counter and attenuation measurements. All measurements were carried out at room temperature. Frequency-dependent attenuation measurements were performed to assess the acoustic response using a method initially described by de Jong et al (de Jong et al. Ultrasonics 1992, 30, 95-103). For this, a narrowband pulse-echo approach was employed, similar to the configuration reported in Goertz et al (Goertz et al. Ultrasound Med Biol 2007, 33, 1376-1388). Two transducers (Model #595396, 5 MHz, 76 mm focus, 12.7 mm diameter, Olympus NDT Canada Inc.; Model #ISO2002HR, 20 MHz, 38 mm focus, 6.35 mm diameter, Valpey-Fisher) were used to cover the frequency range 1.5-27 MHz. The transducers were situated within a water bath and their beams passed through a sample chamber containing diluted agent through a mylar window and were focused upon aluminum reflectors (Supporting Figure 2). An arbitrary waveform generator (Model AWG5002C, Tektronix) was used to generate pulses with center frequencies spaced at 0.5 MHz intervals. The input waveforms were then amplified by 53 dB (Model A-150, ENI) and then sent to one of the two transducers. On receipt, the echoes were amplified by 35 dB (Model AU1583, Miteq), band-pass filtered and then digitized (400 MHz sampling frequency, Agilent Technologies Inc.) for offline analysis. The peak negative pressure at the focus for all waveforms was calibrated to be 25 kPa using a 0.075 mm diameter needle tip hydrophone (Model 1544, Precision Acoustics). Experiments were conducted with agent diluted (1:7500) in saline (0.9% NaCl) and acoustic measurements commenced at the 1 min following dilution. Experiments were conducted once per sample for a total of 3 samples. Size measurements were performed immediately after having extracted the agent from the vial, and attenuation experiments were performed following the Coulter counter measurements using samples from the same vial extraction. Microbubble shell properties were estimated using the general approach initially described in de Jong et al (N. de Jong, L. Hoff, T. Skotland, N. Bom, Ultrasonics 1992, 30, 95-103.). The specific details of the approach employed here are discussed elsewhere (D. E. Goertz, N. de Jong, A. F. van der Steen, Ultrasound Med Biol 2007, 33, 1376-1388.).

### In vivo microbubble ultrasound imaging of the mouse jugular vein

Animal experiments were conducted in accordance with University Health Network guidelines. Porphyrin-lipid microbubbles were imaged in the jugular vein of female BALB/cJ mice. Hair was removed from the neck of the mouse using a chemical hair remover (Nair; Carter-Horner). The mouse was laid supine on a platform with all legs taped to ECG electrodes for heart rate and respiratory monitoring. The mouse was heated on the plateform for approximately 5minutes to dilate its blood vessels. A 27G needle was used to inject a 30µl bolus of microbubbles (∼ 1.5x10⁹ bubbles/ml) via tail vein. The jugular vein was imaged using the VisualSonics Vevo2100 scanner equipped with an 18MHz transducer in contrast mode. Images were acquired before the injection of porphyrin-lipid microbubbles and 4s after injection. Image contrast was auto-adjusted linearly.

### In vivo microbubble ultrasound imaging of a subcutaneous mouse tumour

A NIH swiss female athymic nude mouse bearing a subcutaneous MDA-MB-231 tumour in the left flank was injected via the tail vein using a 26 gauge indwelling catheter with a 50µl bolus of ∼1.5x10⁹ porshe microbubbles/ml followed by a 150 µl saline flush. The mouse was age 12-14 weeks at the time of injection. The tail was warmed using a heated saline pouch to dilate the tail vein prior to catheterization. The tumour was imaged using the Philips iU22 xMATRIX ultrasound system (Philips Medical Systems) and the L12-5 probe with an operating frequency of 5-12 MHz and a mechanical index of 0.07 during simultaneous contrast mode and B-mode images.

### Photoacoustic and ultrasound imaging of microbubbles in a phantom

Photoacoustic characterization was completed using a wavelength-tunable-optical parametric oscillator (OPO) laser (Surelite OPO PLUS; Continuum; wavelength tunability: 650 to 1060 nm) pumped by a Q-switched Nd:YAG laser which was employed to provide laser pulses with a width of 5 ns and a repetition rate of 10 Hz. When the collimated laser pulse traveled through a homemade axicon lens, a ring-shaped light beam was formed. This was focused through an optical condenser and coaxially aligned with the ultrasound focal zone in the water tank. Generated photoacoustic waves were detected by a single-element 10 MHz transducer (V315; Panametrics-NDT), with axial and transverse resolutions of 125 µm and 140 µm, respectively. A sample container was positioned underneath the water tank with an optically and acoustically transparent window covered by a thin transparent membrane coupled with ultrasound gel. Aqueous samples filled the wells that were 9 mm diameter and 3 mm deep. The detected PA signals were first amplified by a low-noise amplifier (5072PR, Panametrics-NDT) and recorded by a digital oscilloscope (TDS 5054, Tektronix). For ultrasound imaging, we utilized the same transducer and raster scanning system as for photoacoustic imaging. In the ultrasound mode, the low-noise amplifier served as both an ultrasound pulse transmitter and receiver. The photoacoustic and ultrasound generation were measured and imaged from three samples: standard microbubbles (0% porphyrin-lipid), porshe microbubbles (15% porphyrin-lipid) and standard microbubbles co-incubated with the same molar concentration of pyro acid as present in 15% porphyrin-lipid microbubbles.

### Results

Here we describe a new class of microbubbles composed with a porphyrin-phospholipid shell encapsulating a gas, preferably a fluorinated gas. These porphyrin shell microbubbles "porshe microbubbles" can be easily synthesized using a standard shaking method (Figure 1) and had a variety of unique physical properties.

When porphyrin-phospholipids were substituted into a standard microbubble formulation at certain concentrations (5-20 molar% porphyrin-phospholipid generated the most bubbles), the resulting microbubbles were found to be formed in higher yield (Figure 2). Without being bound by any theory, we hypothesize that the porphyrin components stabilize the shell and further prevent gas dissolution, resulting in a greater yield; however, some regular phospholipid was necessary to maintain the high yield. A 15 mol % porphyrin-lipid formulation encapsulating perfluoropropane gas was chosen for further studies, as these MBs were formed in high yield with a relatively large amount of incorporated porphyrin-lipid.

The properties of porshe MBs were compared to MBs formed in PBS without porphyrin-lipid, using only DSPC and PEG40S. To evaluate the influence of the porphyrin-lipid on the MB stability, MBs formed without porphyrin-lipid and porshe MBs were incubated in fetal bovine serum at 37 °C. Optical absorbance due to light scattering was used as a measure of the MB concentration and was monitored over a period of 4 h. Porshe MBs displayed much higher stability than MBs without porphyrin-lipid, remaining nearly twice as long in serum (Figure 3), further demonstrating that porphyrin-lipid was not only incorporated into the MB shell but also resulted in a more stable MB. Current clinically used microbubbles typically persist for mere minutes in serum in vivo. The longer survival (nearly 2x) of porshe microbubbles, over regular microbubbles in serum, further suggests that porphyrin-lipid stabilizes the microbubble shell. Any lengthening of circulation times is highly desirable.

It is recognized that monodispersed microbubbles generate a superior ultrasound signal in comparison to polydispersed microbubble populations [12]. Our porphyrin shell microbubbles were shown to have superior monodispersity (Figure 4). High monodispersity is a microbubble property that is highly desirable yet difficult to obtain. This level of monodispersity was only observed when porphyrin-lipid was incorporated into the shell. It is notable that this monodispersed size population was achieved with a simple fabrication method with no further purification step.

The narrow volumetric size distribution of porshe MBs produced a sharp rise in US attenuation with increasing frequency, which exhibited a peak at 9-10 MHz and gradually tapered off by 27.5 MHz (Figure 5). having a higher resonance frequency (f res) than some commercial lipid-based MBs, porshe MBs may be well-suited for imaging applications exploiting the 5-15 MHz frequency range, including breast imaging, carotid neovascularization, superficial tumors, and endoscopic imaging.

The mechanical properties of porshe microbubbles were measured using an acoustic theoretical model introduced by de Jong et al [13] and input data from the acoustic attenuation (Figure 5) and the size distribution (Figure 4). Estimates of the shell stiffness have been previously reported for several commercial lipid MBs, and the porshe MBs were found to be 3-5 times stiffer than these agents (Figure 6), suggesting that a stiffer shell results in a more stable microbubble as a result of decreased gas permeability and a greater resistance to collapse.

The porphyrin shell microbubbles are also intrinsically suited for multimodal imaging. A large number of porphyrins are integrated into the porphyrin shell (over 1 million porphyrins for a typical 15 mol % microbubble composition). This makes them suitable for photoacoustic imaging methods, hybrid PET or MR imaging approaches, or new ultrasound guided optical imaging techniques.

Porshe MBs maintained the nonlinear properties associated with lipid MBs and provided ultrasound contrast. Porshe microbubbles were able to distinguish the jugular vein in a healthy mouse (Figure 7) and image a subcutaneous tumour in a mouse bearing a human breast cancer (MDA-MB-231) xenograft after intravenous injection (Figure 8).

The presence of porphyrin-lipid enables porshe MBs to generate a photoacoustic signal that peaks at 700 nm (Figure 9). MBs formed without porphyrin-lipid, porshe MBs, and MBs without porphyrin-lipid coincubated with equimolar free porphyrin (pyropheophorbide) were photoacoustically imaged in sample wells using a 700 nm, 5 ns pulsed laser and a 10 MHz single-element US transducer (Figure 10a top). The porshe MBs generated a 10-fold greater PA signal than MBs formed without porphyrin-lipid and a 6-fold greater PA signal than MBs without porphyrin-lipid mixed with free porphyrin (Figure 10b). This demonstrates not only that the porphyrin must be present in order for the MBs to generate a PA signal but also that the porphyrin must be conjugated to the lipid and present within the MB shell. All of the MB populations generated strong US signals (Figure 10a bottom, Figure 10b), confirming that porshe MBs have the capacity to serve as a dual-modality US/PA contrast agent.

Bursting of the porphyrin-lipid microbubbles can be accomplished using high power ultrasound or sonication. Upon microbubble bursting, small porphyrin-lipid assemblies (nanometer scale) would be formed which may or may not maintain the close packing density of the porphyrin-lipid. Bursting of the porphyrin-lipid microbubbles could be useful in applications relating to medical imaging and drug delivery by externally triggered delivery of the smaller porphyrin-lipid assemblies to soft tissue.

These independent but complementary advantageous traits make porshe microbubbles ideally-suited to become an improved imaging agent for both existing ultrasound imaging, as well as emerging multimodal imaging approaches (e.g. photoacoustic imaging).

### References

[1] S. Liu and M. A. Vannan, "Use of Echo Contrast in Routine Practice," in Case Based Echocardiography, T. Abraham, Ed. London: Springer London, 2010, pp. 117-125.
[2] F. Moriyasu and K. Itoh, "Efficacy of Perflubutane Microbubble-Enhanced Ultrasound in the Characterization and Detection of Focal Liver Lesions: Phase 3 Multicenter Clinical Trial," American Journal of Roentgenology, vol. 193, no. 1, pp. 86 -95, Jul. 2009.
[3] H. P. Weskott, "Emerging roles for contrast-enhanced ultrasound," Clinical Hemorheology and Microcirculation, vol. 40, no. 1, pp. 51-71, Jan. 2008.
[4] A. L. Klibanov, "Ligand-Carrying Gas-Filled Microbubbles: Ultrasound Contrast Agents for Targeted Molecular Imaging," Bioconjugate Chemistry, vol. 16, no. 1, pp. 9-17.
[5] A. Lawrie, A. F. Brisken, S. E. Francis, D. C. Cumberland, D. C. Crossman, and C. M. Newman, "Microbubble-enhanced ultrasound for vascular gene delivery," Gene Therapy, vol. 7, no. 23, pp. 2023-2027, Dec. 2000.
[6] S. Hernot, "Microbubbles in ultrasound-triggered drug and gene delivery," Advanced Drug Delivery Reviews, vol. 60, no. 10, pp. 1153-1166.
[7] M. Kinoshita, N. McDannold, F. A. Jolesz, K. Hynynen, "Noninvasive localized delivery of Herceptin to the mouse brain by MRI-guided focused ultrasound-induced blood-brain barrier disruption," Proceedings of the National Academy of Sciences, vol. 103, no. 31, pp. 11719-11723, Aug. 2006.
[8] M. Kinoshita, N. McDannold, F. A. Jolesz, K. Hynynen, "Targeted delivery of antibodies through the blood-brain barrier by MRI-guided focused ultrasound," Biochemical and Biophysical Research Communications, vol. 340, pp. 1085-1090,
[9] J. R. Lindner, J. Song, J. Christiansen, A. L. Klibanov, F. Xu, and K. Ley, "Ultrasound Assessment of Inflammation and Renal Tissue Injury With Microbubbles Targeted to P-Selectin," Circulation, vol. 104, no. 17, pp. 2107 - 2112, Oct. 2001.
[10] D. M. Skyba, R. J. Price, A. Z. Linka, T. C. Skalak, and S. Kaul, "Direct In Vivo Visualization of Intravascular Destruction of Microbubbles by Ultrasound and its Local Effects on Tissue," Circulation, vol. 98, no. 4, pp. 290 -293, Jul. 1998.
[11] J. F. Lovell et al., "Porphysome nanovesicles generated by porphyrin bilayers for use as multimodal biophotonic contrast agents," Nat Mater, vol. 10, no. 4, pp. 324-332, Apr. 2011.
[12] J. E. Streeter, R. Gessner, I. Miles, and P. A. Dayton, "Improving sensitivity in ultrasound molecular imaging by tailoring contrast agent size distribution: in vivo studies," Molecular Imaging, vol. 9, no. 2, pp. 87-95, Apr. 2010.
[13] de Jong, N.; Hoff, L.; Skotland, T.; Bom, N, "Absorption and scatter of encapsulated gas filled microspheres: theoretical considerations and some measurements," Ultrasonics, vol. 30, no. 2, pp. 95-103, Mar. 1992.

## Claims

1. A monolayer microbubble with a gas encapsulated therein the monolayer comprising porphyrin-phospholipid conjugate, a second phospholipid and a PEGylated emulsifier, wherein
the porphyrin-phospholipid conjugate comprises one porphyrin, porphyrin derivative or porphyrin analog covalently attached to a lipid side chain by a carbon chain linker of 0 to 20 carbons, preferably at the sn-1 or the sn-2 position, of one phospholipid;
the porphyrin-phospholipid conjugate present at up to 40 molar % of the monolayer;
the porphyrin, porphyrin derivative or porphyrin analog in the porphyrin-phospholipid conjugate is selected from the group consisting of hematoporphyrin, protoporphyrin, tetraphenylporphyrin, a pyropheophorbide, a bacteriochlorophyll, chlorophyll a, a benzoporphyrin, a tetrahydroxyphenyl chlorin, a purpurin, a benzochlorin, a naphthochlorins, a verdin, a rhodin, a keto chlorin, an azachlorin, a bacteriochlorin, a tolyporphyrin, a benzobacteriochlorin, texaphyrin, a sapphyrin, hexaphyrin, porphycene, inverted porphyrin, phthalocyanine, and naphthalocyanine; and
the gas is a biologically inert gas, a fluorinated gas, or selected from the group consisting of air, O2, N2, H2, CO2, N2O, noble gases, hydrocarbon gases, perfluorocarbon, sulphur hexafluoride, perfluoromethane, perfluoroethane, perfluoropropane, perfluorobutane and combinations thereof.

2. The microbubble of claim 1 comprising up to 30, or 20 molar % or between 5-20, 5-15, or 10-15 molar %, or preferably 15 molar % porphyrin-phospholipid conjugate.

3. The microbubble of any one of claims 1-2 wherein the porphyrin, porphyrin derivative or porphyrin analog in the porphyrin-phospholipid conjugate is pyropheophorbide-a acid or a bacteriochlorophyll.

4. The microbubble of any one of claims 1-3 wherein a phospholipid in the porphyrin-phospholipid conjugate comprises phosphatidylcholine, phosphatidylethanoloamine, phosphatidylserine or phosphatidylinositol, preferably wherein the phospholipid comprises an acyl side chain of 12 to 22 carbons.

5. The microbubble of any one of claims 1-3 wherein a phospholipid in the porphyrin-phospholipid conjugate is 1-Palmitoyl-2-Hydroxy-sn-Glycero-3-Phosphocholine or 1-Stearoyl-2-Hydroxy-sn-Gycero-3-Phosphocholine or wherein the porphyrin-phospholipid conjugate is pyropheophorbide-lipid or wherein the porphyrin-phospholipid conjugate is oxy-bacteriochlorophyll-lipid.

6. The microbubble of any one of claims 1-5 wherein the PEGylated emulsifier has a molecular weight ranging from 1000 to 5000 or wherein the PEGylated emulsifier is selected from the group consisting of N-(methoxypolyethylene glycol 5000 carbamoyl)-1,2-dipalmitoyl-sn-glycero-3-phosphatidylethanolamine (MPEG5000-DPPE), 1,2-dimyristoyl-sn-glycero-3-phosphoethanolamine-N-[methoxy(polyethylene glycol)-2000 (DMPE-PEG2000), 1,2-distearoyl-sn-glycero-3-phosphoethanolamine- N-[methoxy(polyethylene glycol)-2000 (DSPE-PEG2000), Polyoxyethylene 40 stearate (PEG40S) and combinations thereof.

7. The microbubble of claim 6 wherein the PEG or PEG-lipid is present in an amount of 10 molar %.

8. The microbubble of any one of claims 1-7, wherein the microbubble is substantially spherical and between 0.8 µm - 18µm in diameter, preferably between 1 µm - 9µm in diameter, further preferably between 2 µm - 4µm in diameter.

9. The microbubble of any one of claims 1-8, wherein the porphyrin-phospholipid conjugate comprises a metal chelated therein, optionally a radioisotope of a metal, preferably wherein the metal is selected from the group consisting of Zn, Cu, Mn, Fe and Pd.

10. The microbubble of any one of claims 1-9, wherein the second phospholipid is an anionic phospholipid.

11. The microbubble of any one of claims 1-9, wherein the phospholipid is selected from the group consisting of phosphatidylcholines, phosphatidylethanolamines, phosphatidic acid, phosphatidylglycerols and combinations thereof, or preferably wherein the phospholipid is selected from the group consisting of 1,2-dipalmitoyl-sn-glycero-3-phosphatidic acid (DPPA), 1,2-dipalmitoyl-sn-glycero-3-phosphatidylcholine (DPPC), 1,2-distearoyl-sn-glycero-3-phosphocholine (DSPC), 1,2-dimyristoyl-sn-glycero-3-phosphocholine (DMPC), 1,2-dibehenoyl-sn-glycero-3-phosphocholine (DBPC), 1,2-diarachidoyl-sn-glycero-3-phosphatidylcholine (DAPC), 1,2-dilignoceroyl-sn-glycero-3-phosphatidylcholine(DLgPC), 1,2-dipalmitoyl-sn-glycero-3-[phosphor-rac-(1-glycerol)] (DPPG) and combinations thereof.

12. The microbubble of any one of claims 1-11, wherein the microbubble is formed in solution comprising at least one additive selected from the group consisting of Sodium Chloride, Sodium Phosphate, Propylene glycol, Glycerol, Polyethylene glycol (preferably ranging from 1000 to 6000 molecular weight) and combinations thereof or further comprising a targeting molecule.

13. A method of preparing the microbubble of any one of claims 1-12, comprising mixing the gas, the porphyrin-phospholipid conjugate, the PEGylated emulsifier and the second phospholipid, wherein the porphyrin-phospholipid conjugate comprises one porphyrin, porphyrin derivative or porphyrin analog covalently attached to a lipid side chain, preferably at the sn-1 or the sn-2 position, of one phospholipid, preferably wherein prior to mixing with the gas, the porphyrin-phospholipid conjugate is mixed with the other phospholipid to form a lipid film and then hydrated in buffer.

14. A method of performing ultrasound imaging on a target area in a subject comprising:
a. providing the microbubble of any one of claims 1-12;
b. administering the microbubble to the subject; and
c. imaging the target area using ultrasound.

15. A method of imaging a target area in a subject, comprising
a. providing the microbubble of any one of claims 1-12;
b. administering the microbubble to the subject; and
c. measuring and/or detecting a photoacoustic signal at the target area,
preferably further comprising bursting using ultrasound or sonication the microbubble at the target area before step c.

## Patentansprüche

1. Monolagen-Mikroblase mit einem darin eingekapselten Gas, wobei die Monolage ein Porphyrin-Phospholipid-Konjugat, ein zweites Phospholipid und einen PEGylierten Emulgator umfasst, wobei
das Porphyrin-Phospholipid-Konjugat ein Porphyrin, Porphyrin-Derivat oder Porphyrin-Analogon umfasst, das durch einen Kohlenstoffkettenverknüpfer mit 0 bis 20 Kohlenstoffen kovalent an eine Lipidseitenkette, vorzugsweise an der sn-1 oder der sn-2 Position, eines Phospholipids gebunden ist;
das Porphyrin-Phospholipid-Konjugat zu bis zu 40 Mol-% der Monolage vorhanden ist;
das Porphyrin, Porphyrin-Derivat oder Porphyrin-Analogon in dem Porphyrin-Phospholipid-Konjugat ausgewählt ist aus der Gruppe bestehend aus Hämatoporphyrin, Protoporphyrin, Tetraphenylporphyrin, einem Pyropheophorbid, einem Bakteriochlorophyll, Chlorophyll a, einem Benzoporphyrin, einem Tetrahydroxyphenylchlorin, einem Purpurin, einem Benzochlorin, einem Naphthochlorin, einem Verdin, einem Rhodin, einem Ketochlorin, einem Azachlorin, einem Bakteriochlorin, einem Tolyporphyrin, einem Benzobakteriochlorin, Texaphyrin, einem Sapphyrin, Hexaphyrin, Porphycen, invertiertem Porphyrin, Phthalocyanin und Naphthalocyanin; und
das Gas ein biologisch inertes Gas, ein fluoriertes Gas ist oder ausgewählt ist aus der Gruppe bestehend aus Luft, O2, N2, H2, CO2, N2O, Edelgasen, Kohlenwasserstoffgasen, Perfluorcarbon, Schwefelhexafluorid, Perfluormethan, Perfluorethan, Perfluorpropan, Perfluorbutan und Kombinationen davon.

2. Mikroblase nach Anspruch 1, die bis zu 30 oder 20 Mol-% oder 5-20, 5-15 oder 10-15 Mol-% oder vorzugsweise 15 Mol-% Porphyrin-Phospholipid-Konjugat umfasst.

3. Mikroblase nach einem der Ansprüche 1-2, wobei das Porphyrin, Porphyrin-Derivat oder Porphyrin-Analogon in dem Porphyrin-Phospholipid-Konjugat Pyropheophorbid-a-Säure oder ein Bakteriochlorophyll ist.

4. Mikroblase nach einem der Ansprüche 1-3, wobei ein Phospholipid in dem Porphyrin-Phospholipid-Konjugat Phosphatidylcholin, Phosphatidylethanoloamin, Phosphatidylserin oder Phosphatidylinositol umfasst, wobei das Phospholipid vorzugsweise eine Acylseitenkette mit 12 bis 22 Kohlenstoffen umfasst.

5. Mikroblase nach einem der Ansprüche 1-3, wobei ein Phospholipid in dem Porphyrin-Phospholipid-Konjugat 1-Palmitoyl-2-hydroxy-sn-glycero-3-phosphocholin oder 1-Stearoyl-2-hydroxy-sn-glycero-3-phosphocholin ist oder wobei das Porphyrin-Phospholipid-Konjugat Pyropheophorbid-Lipid ist oder wobei das Porphyrin-Phospholipid-Konjugat Oxy-Bakteriochlorophyll-Lipid ist.

6. Mikroblase nach einem der Ansprüche 1-5, wobei der PEGylierte Emulgator eine Molekülmasse im Bereich von 1000 bis 5000 hat oder wobei der PEGylierte Emulgator ausgewählt ist aus der Gruppe bestehend aus N-(Methoxypolyethylenglykol-5000-carbamoyl)-1,2-dipalmitoyl-sn-glycero-3-phosphatidylethanolamin (MPEG5000-DPPE), 1,2-Dimyristoyl-sn-glycero-3-phosphoethanolamin-N-[methoxy(polyethylenglykol)-2000 (DMPE-PEG2000), 1,2-Distearoyl-sn-glycero-3-phosphoethanolamin-N-[methoxy(polyethylenglykol)-2000 (DSPE-PEG2000), Polyoxyethylen-40-stearat (PEG40S) und Kombinationen davon.

7. Mikroblase nach Anspruch 6, wobei das PEG oder PEG-Lipid in einer Menge von 10 Mol-% vorliegt.

8. Mikroblase nach einem der Ansprüche 1-7, wobei die Mikroblase im Wesentlichen sphärisch ist und einen Durchmesser von 0,8 µm - 18 µm, vorzugsweise einen Durchmesser von 1 µm - 9 µm, ferner vorzugsweise einen Durchmesser von 2 µm - 4 µm hat.

9. Mikroblase nach einem der Ansprüche 1-8, wobei das Porphyrin-Phospholipid-Konjugat ein darin chelatisiertes Metall, gegebenenfalls ein Radioisotop eines Metalls, umfasst, wobei das Metall vorzugsweise ausgewählt ist aus der Gruppe bestehend aus Zn, Cu, Mn, Fe und Pd.

10. Mikroblase nach einem der Ansprüche 1-9, wobei das zweite Phospholipid ein anionisches Phospholipid ist.

11. Mikroblase nach einem der Ansprüche 1-9, wobei das Phospholipid ausgewählt ist aus der Gruppe bestehend aus Phosphatidylcholinen, Phosphatidylethanolaminen, Phosphatidsäure, Phosphatidylglycerolen und Kombinationen davon oder wobei das Phospholipid vorzugsweise ausgewählt ist aus der Gruppe bestehend aus 1,2-Dipalmitoyl-sn-glycero-3-phosphatidsäure (DPPA), 1,2-Dipalmitoyl-sn-glycero-3-phosphatidylcholin (DPPC), 1,2-Distearoyl-sn-glycero-3-phosphocholin (DSPC), 1,2-Dimyristoyl-sn-glycero-3-phosphocholin (DMPC), 1,2-Dibehenoyl-sn-glycero-3-phosphocholin (DBPC), 1,2-Diarachidoyl-sn-glycero-3-phosphatidylcholin (DAPC), 1,2-Dilignoceroyl-sn-glycero-3-phosphatidylcholin (DLgPC), 1,2-Dipalmitoyl-sn-glycero-3-[phosphor-rac-(1-glycerol)] (DPPG) und Kombinationen davon.

12. Mikroblase nach einem der Ansprüche 1-11, wobei die Mikroblase in einer Lösung gebildet wird, umfassend wenigstens einen Zusatzstoff, der ausgewählt ist aus der Gruppe bestehend aus Natriumchlorid, Natriumphosphat, Propylenglykol, Glycerol, Polyethylenglykol (vorzugsweise mit einer Molekülmasse im Bereich von 1000 bis 6000) und Kombinationen davon, oder ferner umfassend ein Targeting-Molekül.

13. Verfahren zur Herstellung der Mikroblase nach einem der Ansprüche 1-12, das das Mischen des Gases, des Porphyrin-Phospholipid-Konjugats, des PEGylierten Emulgators und des zweiten Phospholipids beinhaltet, wobei das Porphyrin-Phospholipid-Konjugat ein Porphyrin, Porphyrin-Derivat oder Porphyrin-Analogon umfasst, das kovalent an eine Lipidseitenkette, vorzugsweise an der sn-1 oder der sn-2 Position, eines Phospholipids gebunden ist, wobei das Porphyrin-Phospholipid-Konjugat vorzugsweise vor dem Mischen mit dem Gas mit dem anderen Phospholipid gemischt wird, um einen Lipidfilm zu bilden, und dann in einem Puffer hydratisiert wird.

14. Verfahren zur Durchführung einer Ultraschallbilderzeugung an einem Zielbereich eines Subjekts, das Folgendes beinhaltet:
a. Bereitstellen der Mikroblase nach einem der Ansprüche 1-12;
b. Verabreichen der Mikroblase an das Subjekt; und
c. Abbilden des Zielbereichs unter Verwendung von Ultraschall.

15. Verfahren zum Abbilden eines Zielbereichs eines Subjekts, das Folgendes beinhaltet:
a. Bereitstellen der Mikroblase nach einem der Ansprüche 1-12;
b. Verabreichen der Mikroblase an das Subjekt; und
c. Messen und/oder Erfassen eines photoakustischen Signals am Zielbereich,
und vorzugsweise ferner das Zerplatzenlassen der Mikroblase am Zielbereich unter Verwendung von Ultraschall oder Beschallung vor Schritt c beinhaltet.

## Revendications

1. Microbulle de monocouche avec un gaz encapsulé dans celle-ci, la monocouche comprenant un conjugué porphyrine-phospholipide, un deuxième phospholipide et un émulsifiant PEGylé, dans laquelle
le conjugué porphyrine-phospholipide comprend une porphyrine, un dérivé de porphyrine ou un analogue de porphyrine lié de façon covalente à une chaîne latérale lipidique par un lieur de chaîne carbonée de 0 à 20 carbones, de préférence à la position sn-1 ou sn-2, d'un phospholipide ;
le conjugué porphyrine-phospholipide présent jusqu'à 40 % en moles de la monocouche ;
la porphyrine, le dérivé de porphyrine ou l'analogue de porphyrine dans le conjugué porphyrine-phospholipide est choisi dans le groupe constitué de l'hématoporphyrine, la protoporphyrine, la tétraphénylporphyrine, un pyrophéophorbide, une bacteriochlorophylle, la chlorophylle a, une benzoporphyrine, une tétrahydroxyphénylchlorine, une purpurine, une benzochlorine, des naphtochlorines, une verdine, une rhodine, une cétochlorine, une azachlorine, une bactériochlorine, une tolyporphyrine, une benzobactériochlorine, la texaphyrine, une saphyrine, l'hexaphyrine, le porphycène, une porphyrine inversée, la phtalocyanine et la naphtalocyanine ; et
le gaz est un gaz biologiquement inerte, un gaz fluoré, ou choisi dans le groupe constitué de l'air, O2, N2, H2, CO2, N2O, des gaz nobles, des gaz hydrocarbures, un perfluorocarbure, l'hexafluorure de soufre, le perfluorométhane, le perfluoroéthane, le perfluoropropane, le perfluorobutane et des combinaisons de ceux-ci.

2. Microbulle selon la revendication 1 comprenant jusqu'à 30, ou 20 % en moles ou entre 5 et 20, 5 et 15, ou 10 et 15 % en moles, ou de préférence 15 % en moles de conjugué porphyrine-phospholipide.

3. Microbulle selon l'une quelconque des revendications 1 à 2, dans laquelle la porphyrine, le dérivé de porphyrine ou l'analogue de porphyrine dans le conjugué porphyrine-phospholipide est le pyrophéophorbide a acide ou une bactériochlorophylle.

4. Microbulle selon l'une quelconque des revendications 1 à 3, dans laquelle un phospholipide dans le conjugué porphyrine-phospholipide comprend la phosphatidylcholine, la phosphatidyléthanolamine, la phosphatidylsérine ou le phosphatidylinositol, de préférence dans laquelle le phospholipide comprend une chaîne latérale acyle de 12 à 22 carbones.

5. Microbulle selon l'une quelconque des revendications 1 à 3, dans laquelle un phospholipide dans le conjugué porphyrine-phospholipide est la 1-palmitoyl-2-hydroxy-sn-glycéro-3-phosphocholine ou la 1-stéaroyl-2-hydroxy-sn-glycéro-3-phosphocholine ou dans laquelle le conjugué porphyrine-phospholipide est pyrophéophorbide-lipide ou dans laquelle le conjugué porphyrine-phospholipide est oxy-bactériochlorophylle-lipide.

6. Microbulle selon l'une quelconque des revendications 1 à 5, dans laquelle l'émulsifiant PEGylé a un poids moléculaire dans la plage de 1000 à 5000 ou dans laquelle l'émulsifiant PEGylé est choisi dans le groupe constitué des N-(méthoxypolyéthylène glycol 5000 carbamoyl)-1,2-dipalmitoyl-sn-glycéro-3-phosphatidyléthanolamine (MPEG5000-DPPE), 1,2-dimyristoyl-sn-glycéro-3-phosphoéthanolamine-N-[méthoxy(polyéthylène glycol)-2000 (DMPE-PEG2000), 1,2-distéaroyl-sn-glycéro-3-phosphoéthanolamine-N-[méthoxy(polyéthylène glycol)-2000 (DSPE-PEG2000), stéarate de polyoxyéthylène 40 (PEG40S) et des combinaisons de ceux-ci.

7. Microbulle selon la revendication 6, dans laquelle le PEG ou PEG-lipide est présent en une quantité de 10 % en moles.

8. Microbulle selon l'une quelconque des revendications 1 à 7, la microbulle étant sensiblement sphérique et comprise entre 0,8 µm et 18 µm de diamètre, de préférence entre 1 µm et 9 µm de diamètre, plus préférablement entre 2 µm et 4 µm de diamètre.

9. Microbulle selon l'une quelconque des revendications 1 à 8, dans laquelle le conjugué porphyrine-phospholipide comprend un métal chélaté dans celui-ci, facultativement un radio-isotope d'un métal, de préférence dans laquelle le métal est choisi dans le groupe constitué de Zn, Cu, Mn, Fe et Pd.

10. Microbulle selon l'une quelconque des revendications 1 à 9, dans laquelle le deuxième phospholipide est un phospholipide anionique.

11. Microbulle selon l'une quelconque des revendications 1 à 9, dans laquelle le phospholipide est choisi dans le groupe constitué de phosphatidylcholines, de phosphatidyléthanolamines, de l'acide phosphatidique, de phosphatidylglycérols et des combinaisons de ceux-ci ou, de préférence, dans laquelle le phospholipide est choisi dans le groupe constitué des acide 1,2-dipalmitoyl-sn-glycéro-3-phosphatidique (DPPA), 1,2-dipalmitoyl-sn-glycéro-3-phosphatidylcholine (DPPC), 1,2-distéaroyl-sn- glycéro-3-phosphocholine (DSPC), 1,2-dimyristoyl-sn-glycéro-3-phosphocholine (DMPC), 1,2-dibéhénoyl-sn-glycéro-3-phosphocholine (DBPC), 1,2-diarachidoyl-sn-glycéro-3-phosphatidylcholine (DAPC), 1,2-dilignocéroyl-sn-glycéro-3-phosphatidylcholine(DLgPC), 1,2-dipalmitoyl-sn-glycéro-3-[phosphor-rac-(1-glycérol)] (DPPG) et des combinaisons de ceux-ci.

12. Microbulle selon l'une quelconque des revendications 1 à 11, la microbulle étant formée dans une solution comprenant au moins un additif choisi dans le groupe constitué des chlorure de sodium, phosphate de sodium, propylène glycol, glycérol, polyéthylène glycol (de préférence dans la plage de poids moléculaire de 1000 à 6000) et des combinaisons de ceux-ci ou comprenant en outre une molécule de ciblage.

13. Procédé de préparation de la microbulle selon l'une quelconque des revendications 1 à 12, comprenant le mélange du gaz, du conjugué porphyrine-phospholipide, de l'émulsifiant PEGylé et du deuxième phospholipide, dans lequel le conjugué porphyrine-phospholipide comprend une porphyrine, un dérivé de porphyrine ou un analogue de porphyrine lié de façon covalente à une chaîne latérale lipidique, de préférence à la position sn-1 ou sn-2, d'un phospholipide, de préférence dans lequel, avant mélange avec le gaz, le conjugué porphyrine-phospholipide est mélangé avec l'autre phospholipide pour former un film lipidique, puis hydraté dans un tampon.

14. Procédé de conduite d'une imagerie ultrasonore sur une zone cible dans un sujet comprenant :
a. la fourniture de la microbulle selon l'une quelconque des revendications 1 à 12 ;
b. l'administration de la microbulle au sujet ; et
c. l'imagerie de la zone cible au moyen d'ultrasons.

15. Procédé d'imagerie d'une zone cible dans un sujet, comprenant
a. la fourniture de la microbulle selon l'une quelconque des revendications 1 à 12 ;
b. l'administration de la microbulle au sujet ; et
c. la mesure et/ou la détection d'un signal photoacoustique au niveau de la zone cible,
de préférence comprenant en outre l'éclatement au moyen d'ultrasons ou par sonication de la microbulle au niveau de la zone cible avant l'étape c.
